# EUROPEAN PATENT SPECIFICATION

(11) **EP 2 913 787 B1**
(45) Date of publication and mention of the grant of the patent: **29.09.2021**
(21) Application number: 13848657.6
(22) Date of filing: 22.10.2013
(51) Int. Cl.: G06Q 10/10, G01N 35/00, G06Q 50/22, G01N 35/02, G06Q 10/06, G01N 35/04, G16H 40/20, G16H 40/40

(54) **AUTOMATIC ANALYSIS DEVICE**
AUTOMATISCHE ANALYSEVORRICHTUNG
DISPOSITIF D'ANALYSE AUTOMATIQUE

(30) Priority: 25.10.2012 JP 2012235606
(43) Date of publication of application: 02.09.2015
(73) Proprietor: Hitachi High-Tech Corporation, Minato-ku Tokyo 105-6409 (JP)
(72) Inventor: KANEKO, Yasuo, Tokyo 105-6409 (JP); SAITO, Yoshiaki, Tokyo 105-6409 (JP)
(74) Representative: Strehl Schübel-Hopf & Partner
(86) International application number: PCT/JP2013/078576
(87) International publication number: WO 2014/065277

(56) References cited:
- JP-A- 2002 083 101
- JP-A- 2002 083 101
- JP-A- 2003 256 384
- JP-A- 2004 094 511
- JP-A- 2006 004 021
- JP-A- 2006 338 485
- JP-A- 2007 241 393
- JP-A- 2008 059 523
- JP-A- 2008 059 523
- JP-A- 2008 066 633
- JP-A- 2009 168 730
- JP-A- 2009 270 841
- JP-A- 2011 022 734
- US-A1- 2008 050 280

## Description

### Technical Field

The present invention relates generally to automatic analyzers that measure biological samples such as blood and urine and, more particularly, to an automatic analyzer that includes a user interface for efficiently planning and controlling a task schedule for a user in the automatic analyzer.

### Background Art

Automatic analyzers that perform analysis using a biological sample, such as blood and urine, and a reagent require calibration as an operation to calibrate a calibration curve and accuracy control for allowing the automatic analyzer to maintain its best possible condition at the start of analysis or at predetermined intervals established according to each reagent or as necessary during an analysis process.

Examples of factors mandating the calibration or accuracy control include, but are not limited to, the lapse of a predetermined period of time for periodically performing the calibration or accuracy control, after a change of reagent bottles, and re-measurement following a measurement failure.

Calibration is performed using a standard solution having a concentration established according to each item. The calibration or the accuracy control has its own validity period established according to each analysis item. Expiration of the validity period thus calls for new calibration or new accuracy control. The new calibration or the new accuracy control also needs to be performed when, in each analysis item, the reagent in one reagent bottle is used up and a reagent in a new reagent bottle is to be used.

A known automatic analyzer automatically detects, for example, the lapse of the predetermined period of time established according to each reagent, the expiration of the validity period, and the changeover from one reagent bottle to another and notifies the user that the calibration or the accuracy control needs to be performed (see, for example,
Patent Document 1).
Further, JP 2009 168730 A discloses an automatic analyzer which displays scheduled dates and times for future calibration and quality control of analytes. Related art is disclosed in US 2008/050280 A1, JP 2008 059523 A, JP 2002 083101 A and JP 2009 270841 A.

### Prior Art Documents

### Patent Document

### Patent Document 1

JP-2006-53164-A

### Summary of the Invention

### Problem to be Solved by the Invention

The function of the technique disclosed in patent document 1 is indeed effective in indicating the necessity for calibration or accuracy control for each analysis item immediately at a particular point in time. It is, however, an impending moment when the user knows the necessity for the performance with respect to any one of the factors. No means have so far been available for predicting the necessity for the calibration or accuracy control and preparing an analysis schedule with respect to a predetermined period of time in the future. It has thus been difficult for the user to prepare a standard calibration sample, an accuracy control sample, and reagents for analyzing these samples, and perform analysis according to a planned schedule.

Recent years have witnessed devices offering higher precision and higher sensitivity to achieve improved performance of the automatic analyzer, which is accompanied by an increase in the number of maintenance functions for maintaining and controlling the automatic analyzer. An example of the maintenance functions includes necessity for replacing a light source of a photometer incorporated in an automatic analyzer at predetermined intervals. Such a maintenance function requires periodic performance and known automatic analyzers did have a function of controlling a time limit. As with the calibration and accuracy control mentioned above, however, it was the last moment when the user knew the necessity for the performance and it has been difficult to perform maintenance on a scheduled basis.

Furthermore, many of laboratories using automatic analyzers are operated for 24 hours and a plurality of users works in shifts to operate the laboratories. It is thus important for each individual user to perform each and every task event occurring during a period of time allocated to him or her and to transfer his or her duties to the subsequent user.

The known automatic analyzer, however, had no means of inputting a user's work schedule. The known automatic analyzer simply notifies the user of an event at irregular intervals not operatively associated with the user's work schedule. The automatic analyzer's inability to efficiently schedule tasks and let the user perform the tasks on a scheduled basis can cause omission of a task, posing a serious problem impeding assurance of performance.

A large-scale testing facility that includes a large number of analyzers to be operated by users and a plurality of users working simultaneously involves occurrence of omission of a task, leading to an even greater possibility of inefficient work.

It is therefore an object of the present invention to provide an automatic analyzer that enables specific tasks to be performed by a user within a period of time allocated to him or her to be predicted in advance, the tasks to be efficiently scheduled according to a work schedule of the user, and each and every task to be performed without any omission.

### Means for Solving the Problem

To achieve the foregoing object, the present invention provides an automatic analyzer in accordance with claim 1. The dependent claims relate to preferred embodiments of the invention.

The arrangements enable specific tasks to be performed by a user within the period of time allocated to him or her to be predicted in advance, the tasks to be efficiently scheduled according to the work schedule for the user, and each and every task to be performed without any omission.

### Effect of the Invention

The present invention enables specific tasks to be performed by a user within a period of time allocated to him or her to be predicted in advance, the tasks to be efficiently scheduled according to a work schedule of the user, and each and every task to be performed without any omission.

### Brief Description of the Drawings

Fig. 1 is a system configuration diagram showing a general configuration of an automatic analyzer according to an embodiment of the present invention.
Fig. 2 is a block diagram showing a configuration of an operator unit PC incorporated in the automatic analyzer according to the embodiment of the present invention.
Fig. 3 is an explanatory drawing illustrating an exemplary task schedule screen in the automatic analyzer according to the embodiment of the present invention.
Fig. 4 is an explanatory drawing illustrating an exemplary task schedule screen after tasks have been performed and reserved in the automatic analyzer according to the embodiment of the present invention.
Fig. 5 is an explanatory diagram illustrating a flow of data relating to a task schedule control function in the automatic analyzer according to the embodiment of the present invention.
Fig. 6 is an explanatory drawing illustrating an exemplary work schedule input screen in the automatic analyzer according to the embodiment of the present invention.
Fig. 7 is an explanatory drawing illustrating an exemplary task schedule control setting screen in the automatic analyzer according to the embodiment of the present invention.
Fig. 8 is a flowchart showing steps of task schedule preparing and control processes in the automatic analyzer according to the embodiment of the present invention.
Fig. 9 is an explanatory drawing illustrating an exemplary task notifying dialog screen in the automatic analyzer according to the embodiment of the present invention.

### Modes for Carrying Out the Invention

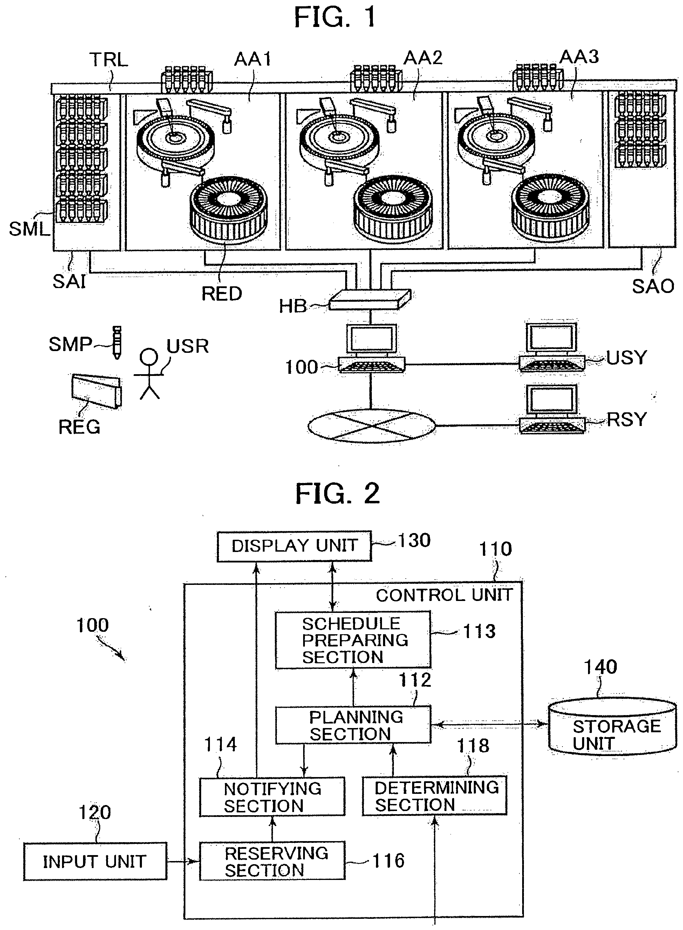

A configuration and operations of an automatic analyzer according to an embodiment of the present invention will be described below with reference to Figs. 1 to 9.

A general configuration of the automatic analyzer according to the embodiment will first be described with reference to Fig. 1.

Fig. 1 is a system configuration diagram showing a general configuration of the automatic analyzer according to the embodiment of the present invention.

The automatic analyzer according to the embodiment includes, for example, three analyzing units AA1, AA2, and AA3 connected in series with each other. The automatic analyzer, though including three analyzing units in this embodiment, may include any other numbers of analyzing units including one. The automatic analyzer further includes a transfer line TRL that transfers sample racks. The transfer line TRL is disposed adjacent to each of the analyzing units AA1, AA2, and AA3. Additionally, the automatic analyzer includes a sample loading unit SAI disposed on a first end portion side of each of the analyzing units AA1, AA2, and AA3 and a sample storing unit SAO disposed on a second end portion side.

A user USR mounts a sample SMP to be subjected to calibration measurement, accuracy control sample measurement, and patient sample measurement on a sample rack SML. A plurality of samples can be mounted on the sample rack SML. The sample rack SML on which samples are mounted is disposed at the sample loading unit SAI. The sample racks SML thus disposed are loaded in sequence onto the transfer line TRL and conveyed onto the analyzing units AA1, AA2, and AA3 that perform analysis on the samples. The analyzing units AA1, AA2, and AA3 each recognize the sample rack SML and the sample SMP and perform analysis required for the sample SMP. The analyzing units AA1, AA2, and AA3 each include a reagent disk RED that houses a plurality of reagents REG used for performing analysis. The reagents REG required for the analysis are loaded in the reagent disk RED by the user in advance of the analysis. The sample rack SML that has undergone the required analysis is conveyed onto the sample storing unit SAO.

The sample loading unit SAI, the analyzing units AA1, AA2, and AA3, and the sample storing unit SAO are each connected through a network cable to an operator unit PC 100 of the automatic analyzer via a hub HB. The operator unit PC 100 is connected to a host system USY over a network.

The user makes an analysis request (measurement request) for each sample from the host system USY or the operator unit PC 100 and mounts the sample to be analyzed on the sample rack SML and loads the sample rack SML onto the sample loading unit SAI. Information of the analysis request is transmitted to the analyzing units AA1, AA2, and AA3 over the network and analyses by the analyzing units AA1, AA2, and AA3 are transmitted to the operator unit PC 100 and the host system USY.

The operator unit PC 100 is also connected to a remote system RSY via a public network. The remote system RSY stores information, such as analysis parameters that represent analysis condition information using reagents to be used in the automatic analyzer. The remote system RSY delivers the analysis parameters upon request from the operator unit PC 100. The delivered analysis parameters are stored in the operator unit PC 100 and transmitted to the analyzing units AA1, AA2, and AA3 during analysis of samples.

A task schedule control function according to the embodiment can achieve the best possible result from standpoints of system configuration and operability when the operator unit PC 100 includes a screen interface. The operator unit PC 100 creates task schedule information for each person in charge using work schedule information input from the operator unit PC 100 or the host system USY or the remote system RSY that assumes an upper system of the automatic analyzer and task event information of the automatic analyzer controlled by the operator unit PC 100.

The user can check specific tasks to be performed by him or her on a task schedule screen displayed on the operator unit PC 100 to thereby formulate an efficient task plan in line with a work schedule.

Alternatively, the host system or the remote system as an upper system may include a task schedule control unit according to a use mode of the user, thereby achieving the function through an exchange of information via the network between the operator unit PC and the analyzing unit.

A configuration of the operator unit PC 100 incorporated in the automatic analyzer according to the embodiment will be described below with reference to Fig. 2.

Fig. 2 is a block diagram showing a configuration of the operator unit PC incorporated in the automatic analyzer according to the embodiment of the present invention.

The operator unit PC 100 mainly includes a control unit 110, an input unit 120, a display unit 130, and a storage unit 140. The input unit 120 includes a keyboard and a mouse. The input unit 120 allows a button displayed on the display unit 130 to be depressed (clicked with the mouse) and a numeral or a character to be input into an input field displayed on the display unit 130. The input unit 120 and the display unit 130 constitute a graphical user interface (GUI).

The control unit 110 includes a planning section 112, a schedule preparing section 113, a notifying section 114, a reserving section 116, and a determining section 118. Functions and operations of these sections will be described later.

The storage unit 140 stores the work schedule information and the task event information. The planning section 112 uses the foregoing information to prepare a task schedule.

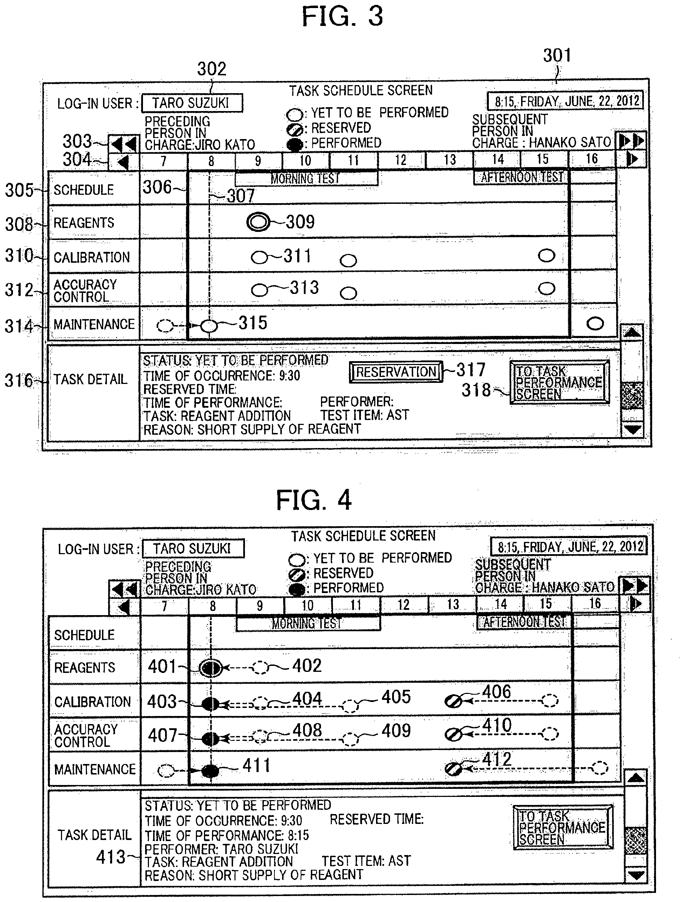

An exemplary task schedule screen in the automatic analyzer according to the embodiment will be described below with reference to Fig. 3.

Fig. 3 is an explanatory drawing illustrating an exemplary task schedule screen in the automatic analyzer according to the embodiment of the present invention.

A task schedule screen 301 is displayed on the display unit 130 shown in Fig. 2. The task schedule screen 301 displays as a time schedule a list of tasks to be performed by the user who has logged in to the operator unit PC 100 within a period of time allocated to him or her.

A log-in user 302 field displays the name of the user who has currently logged in. By default, the task schedule screen 301 displays the task schedule of the log-in user during a corresponding time slot at the center thereof, making the display optimum for the operating user.

Person-in-charge advance/retreat buttons 303 are used to display the task schedule for persons-in-charge who precede and follow the log-in user. Time advance/retard buttons 304 are used to scroll up and down the display of the task schedule by the hour. Using the person-in-charge advance/retreat buttons 303 and the time advance/retard buttons 304, the user can check not only the task schedule of him or her own, but also statuses of tasks of the preceding person in charge and tasks scheduled for the subsequent person in charge.

A schedule display area 305 displays schedules of a hospital and a laboratory. A reagent task display area 308 displays a reagent task event 309, such as a reagent addition necessitated as a result of a short reagent supply. A calibration task display area 310 displays a calibration task event 311, such as calibration measurement that accompanies a reagent addition or a timeout. An accuracy control task display area 312 displays an accuracy control task event 313, such as accuracy control measurement that accompanies a reagent addition or a timeout. A maintenance task display area 314 displays a maintenance task event 315, such as maintenance performance that accompanies a timeout or a part replacement. Preferably, to enhance visibility, a mark that represents each of these task events is changed according to the status of performance of the task.

Additionally, the task schedule screen 301 displays an allocated time frame 306 of the log-in user and a current time of day line 307.

The foregoing display allows the person in charge to readily check the tasks to be performed within the period of time allocated to him or her and to confirm a specific timing at which each of the tasks in the schedules of the hospital and the laboratory occurs, thereby enabling the person in charge to determine whether the tasks can be performed efficiently.

The type of the task events displayed on the screen is optimally concerned with the most critical reagent, calibration, accuracy control, and maintenance in terms of assurance of performance as the automatic analyzer, as exemplified on the exemplary screen; preferably, however, the type of the task events can be customized according to the purpose of the user. In the display example shown in Fig. 3, it is known that the reagent task event 309, the calibration task event 311, the accuracy control task event 313, and the like can be predicted to occur during the morning test, so that the morning test is likely to be interrupted. It is noted that the maintenance task event 315, displayed by a broken-line circle and a solid-line circle connected to each other, indicates that the task not performed by the preceding person in charge is carried over to the log-in user.

A task detail display area 316 displays details of the task event selected on the screen. Fig. 3 shows an exemplary condition in which the reagent task event 309 is selected. In Fig. 3, the reagent task event 309 in the reagent task display area 308 is encircled by a thick-line ring, indicating that reagent task event 309 is exemplarily selected. In actual screens, the circle mark of, for example, a selected event is colored differently from others, indicating that the event is selected.

The task detail display area 316 displays task performance status, occurrence time of day, reserved time of day, performance time of day, performer, and task detail. The task detail display area 316 includes a task reserving button 317 used for reserving a task in advance at a convenient time of day when the occurrence time of day falls at an inconvenient time for reasons of scheduling as described above. The task detail display area 316 further includes a task performance screen shift button 318 that allows each task performance screen for performing a corresponding task to be directly accessed for quick performance of the task. Automatically displaying the task schedule screen 301 as described above upon a user's login to the operator unit PC 100 enables the user to check the specific task to be performed within the period of time allocated to him or her, thereby formulating an efficient task plan. In addition, automatically displaying the task schedule screen 301 upon the user's log-out from the operator unit PC 100 enables the user to check that none of the tasks are left undone during the period of time allocated to him or her.

The following describes with reference to Fig. 4 an exemplary task schedule screen after tasks have been performed and reserved in the automatic analyzer according to the embodiment of the present invention.

Fig. 4 is an explanatory drawing illustrating the exemplary task schedule screen after tasks have been performed and reserved in the automatic analyzer according to the embodiment of the present invention.

In the display example shown in Fig. 3, it is known that the reagent task event, the calibration task event, the accuracy control task event, and the like can be predicted to occur during the morning test, so that the morning test is likely to be interrupted.

In contrast, the user performs each of.a reagent task event 402, calibration task events 404 and 405, accuracy control task events 408 and 409, and a maintenance task event 411 prior to the morning test. This results in a display of "performed" statuses, such as a reagent task event 401, a calibration task event 403, an accuracy control task event 407, the maintenance task event 411, and a task detail display area 413. In addition, connected lines and broken lines are used to facilitate a ready understanding of correspondence to the task events 402, 404, 405, 408, and 409 originally displayed at the occurrence time of day. Specifically, a task indicated by a broken-line circle (e.g., the task event 402) is displayed as a solid-black circle, performed task (e.g., the task event 401) and the broken-line circle and the solid-black circle are connected to each other by a broken-line arrow.

In addition, to ensure that the calibration task event, the accuracy control task event, and the maintenance task event that can be predicted to occur during the afternoon test can be performed in advance of the afternoon test, tasks are reserved as with task events 406, 410, and 412 prior to the afternoon test. Specifically, a task indicated by a broken-line circle is displayed as a hatched circle (e.g., the task event 406) and the broken-line circle and the hatched circle are connected to each other by a broken-line arrow. Performance of a task to be performed during the period of time allocated to the subsequent person in charge, such as the task event 412, in particular, can be expected to achieve a benefit of, for example, reducing a workload on the subsequent person in charge particularly when, for example, the subsequent person in charge is a novice at the automatic analyzer.

Additionally, a comprehensive schedule control not only for the task events on the automatic analyzer but also in working can be achieved by a function that allows the user to register any desired schedule provided in the schedule display area 305 shown in Fig. 3. The display and task reserving functions as described above enables task performance statuses to be readily checked and an efficient task plan to be formulated in line with the work schedule.

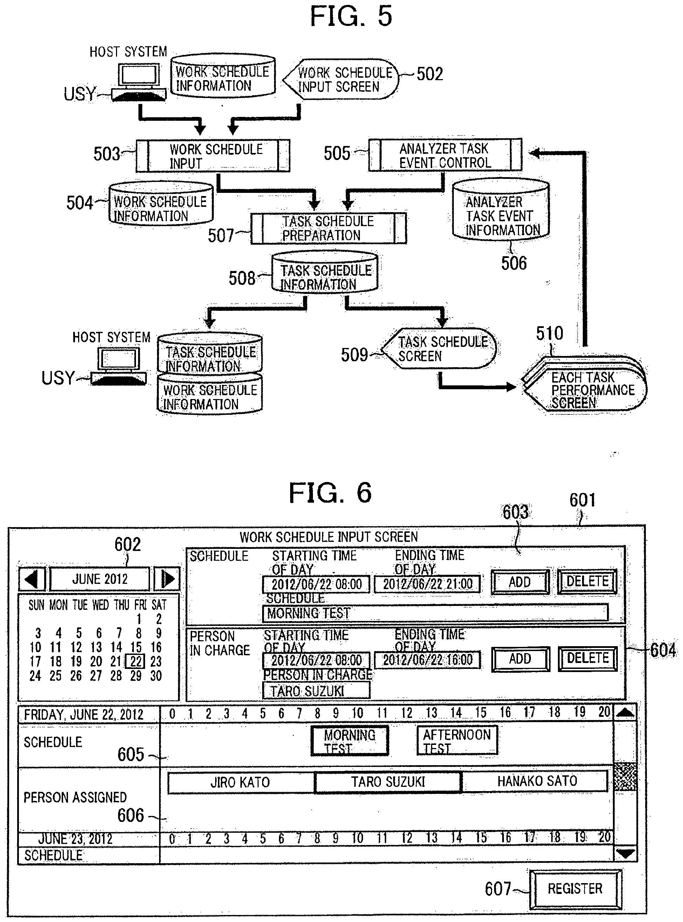

The following describes with reference to Fig. 5 a flow of data relating to a task schedule control function in the automatic analyzer according to the embodiment of the present invention.

Fig. 5 is an explanatory diagram illustrating a flow of data relating to the task schedule control function in the automatic analyzer according to the embodiment of the present invention.

A work schedule input process 503 creates work schedule information 504 through a reception from the host system USY connected to the network or an input from a work schedule input screen 502 on the operator unit PC 100.

An analyzer task event control process 505 creates and controls analyzer task event information 506 relating to the reagent, calibration, accuracy control, and maintenance tasks that can be predicted to occur from parameter and reagent information registered in the analyzer and measurement and maintenance records.

A task schedule preparing process 507 creates task schedule information 508 for each person in charge on the basis of the work schedule information 504 and the analyzer task event information 506. The created task schedule information 508 is displayed on a task schedule screen 509. Additionally, the task schedule information 508 is transmitted to the host system USY connected to the network.

The user checks the task on the task schedule screen and accesses each task performance screen 510 to perform each task. The performed task event is fed back to the analyzer task event control process 505 and the analyzer task event information 506 is then updated.

The following describes a correspondence between the elements shown in Fig. 2 and those shown in Fig. 5.

The planning section 112 shown in Fig. 2 schedules in advance the task events required for using the analyzer, performing the analyzer task event control process 505 shown in Fig. 5.

The schedule preparing section 113 shown in Fig. 2 prepares, on the basis of the work schedule and task events, a time-series task schedule chart that represents a list of tasks to be performed by the user within the period of time allocated to him or her. The schedule preparing section 113 performs the task schedule preparing process 507 shown in Fig. 5.

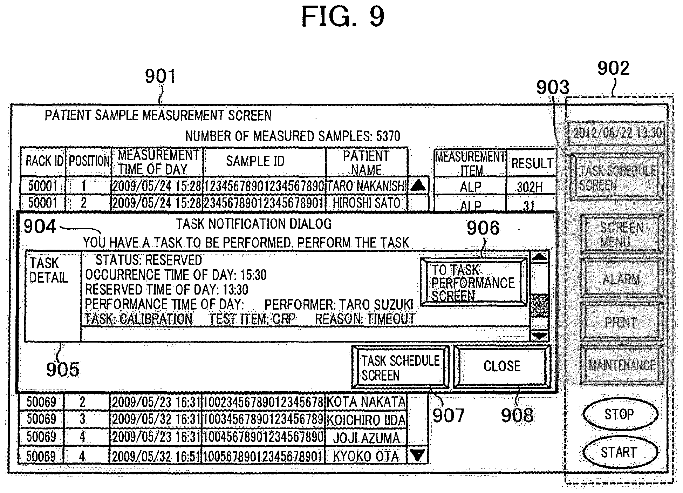

The notifying section 114 shown in Fig. 2 prompts the user to perform a task when a reserved time of day arrives by way of a task notifying dialog screen 904 to be described later with reference to Fig. 9.

The reserving section 116 shown in Fig. 2 allows the user to set any scheduled performance time of day for a task event on the task schedule. Data input by an operator is set through depression of the task reserving button 317 shown in Fig. 3.

The determining section 118 shown in Fig. 2 recognizes the task performed on the analyzer to thereby automatically determine whether a task event displayed on the task schedule chart has been, or has yet to be, performed. The determining section 118 performs the analyzer task event control process 505 shown in Fig. 5.

The input unit 120 shown in Fig. 2 allows a work schedule of a user who uses the analyzer to be input. As described earlier, the input unit 120, and the display unit 130 that displays whether tasks have been performed or not on the task schedule chart, constitute the GUI.

The storage unit 140 shown in Fig. 2 stores the work schedule information 504, the analyzer task event information 506, and the task schedule information 508 shown in Fig. 5.

The following describes an operation of each element with reference to a specific example.

The schedule preparing section 113 prepares, on the basis of the work schedule and task events, a time-series task schedule chart that represents a list of tasks to be performed by the user within the period of time allocated to him or her. The prepared task schedule chart is displayed, for example, on the task schedule screen 301 shown in Fig. 3. For example, a person in charge who has logged in views the task schedule screen 301 and depresses the task performance screen shift button 318 in an attempt to perform the reagent task event 309. This causes the display screen on the display unit 130 to display a procedure for a reagent addition task. Following the procedure, the person in charge performs the reagent addition task. On completing the reagent addition, the person in charge depresses, for example, a "reagent addition completion" button displayed on the display unit 130. This causes the schedule preparing section 113 to recognize that the task in question has been completed.

Meanwhile, the schedule preparing section 113 recognizes that the task in question is yet to be performed until the "reagent addition completion" button is depressed. It is noted that the determining section 118 shown in Fig. 2 determines, for example, whether a task is yet to be performed.

For example, when the time slot shifts from that of the preceding person in charge to that of the current person in charge when the maintenance task to be performed by the preceding person in charge displayed in the maintenance task display area 314 of Fig. 3 is yet to be performed, the schedule preparing section 113 displays the maintenance task yet to be performed as the maintenance task event 315 of the person in charge in question in the task schedule screen 301 of Fig. 3.

Alternatively, when the reagent task event 402 relating to the reagent addition shown in Fig. 4 is performed ahead of schedule, for example, the task performance screen shift button 318 is depressed and a procedure for the reagent addition task displayed on the display screen of the display unit 130 is followed and performed. When the reagent addition is then completed, the "reagent addition completion" button displayed on the display unit 130 is depressed. This results in the reagent addition task in question being assumed to be completed in the analyzer task event control process 505 shown in Fig. 5, so that the analyzer task event information 506 is updated. Through the task schedule preparing process 507, the schedule preparing section 113 refers to the analyzer task event information 506 to thereby recognize that the task in question has been completed. The task in question is then displayed as the performed task event 401 of Fig. 4 on the task schedule screen 301.

To reserve a task ahead of the afternoon test in order for the calibration task event 406 shown in Fig. 4 to be performed ahead of the afternoon test, the calibration task event 406 is selected. Then, the detail of the calibration task event 406 is displayed as in the task detail display area 316 of Fig. 3. The person in charge then enters, for example, "8:30" in the "reserved time of day" field and depresses the task reserving button 317. As a result, through the analyzer task event control process 505 shown in Fig. 5, the analyzer task event information 506 is updated to incorporate the changed reserved time of day. Through the task schedule preparing process 507, the schedule preparing section 113 refers to the analyzer task event information 506 to thereby display the revised task schedule on the task schedule screen 301 like the calibration task event 406 of Fig. 4.

The following describes with reference to Fig. 6 an exemplary work schedule input screen in the automatic analyzer according to the embodiment of the present invention.

Fig. 6 is an explanatory drawing illustrating an exemplary work schedule input screen in the automatic analyzer according to the embodiment of the present invention.

A work schedule input screen 601 is used by a user in the capacity of an administrator or equivalent to register a daily test schedule and assignment of persons in charge. The work schedule input screen 601 is mounted on the operator unit PC of the automatic analyzer or the host system connected by the network. Large-scale hospitals and laboratories have a large number of analyzers and a large number of persons in charge, so that it is efficient and ideal to mount the work schedule input screen 601 in the host system thereby achieving integrated control.

A calendar 602 is used for selecting a date of the test schedule and personnel assignment. With respect to the selected date, specific times of day are specified to set up a schedule in a schedule input area 603. The input schedule is displayed in a schedule display area 605.

In addition, times of day is specified and the person in charge is entered in a person in charge input area 604. A personnel assignment display area 606 displays the entered persons in charge assigned.

The input information on the schedule and personnel assignment is saved by the depression of a register button 607. It is noted that a configuration of the work schedule input screen 601 mounted on the host system involves control of a plurality of automatic analyzers. As a result, information for identifying a specific automatic analyzer is added and the screen is laid out to allow a schedule for each individual automatic analyzer to be input.

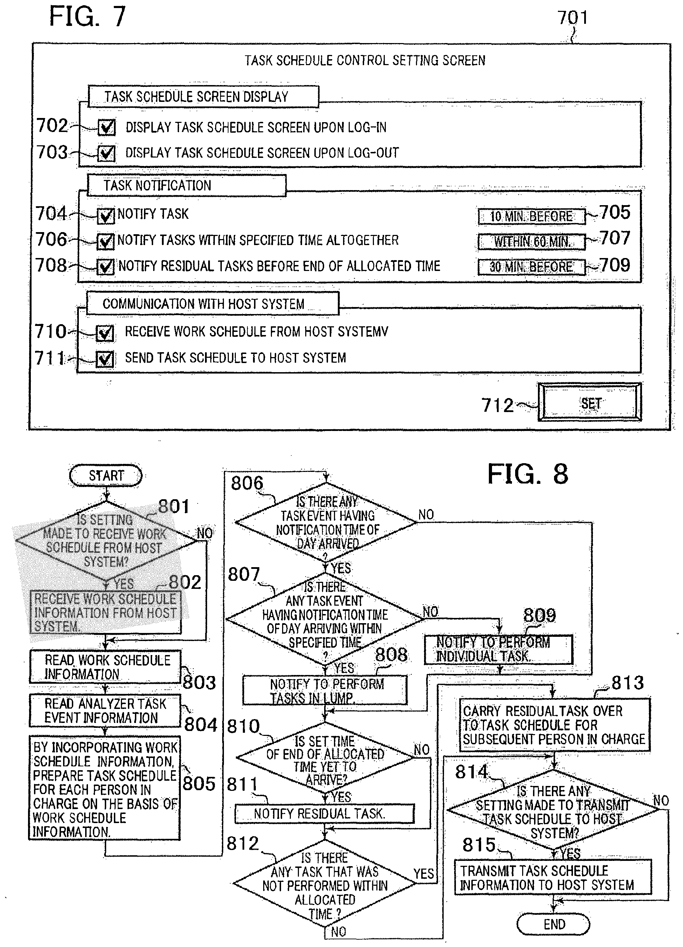

The following describes with reference to Fig. 7 an exemplary task schedule control setting screen in the automatic analyzer according to the embodiment of the present invention.

Fig. 7 is an explanatory drawing illustrating an exemplary task schedule control setting screen in the automatic analyzer according to the embodiment of the present invention.

A task schedule control setting screen 701 provides a screen for setting, for example, screen display, task notification, and communication with the host system in the task schedule control function.

A log-in task schedule display setting field 702 is used to set whether or not to automatically display the task schedule screen of the log-in user when a user logs in to the system on the operator unit PC.

Similarly, a log-out task schedule display setting field 703 is used to set whether or not to automatically display the task schedule screen of the log-in user when the user logs out from the system.

The task schedule screen display function described above allows the user to check the schedule of a specific task he or she is required to perform within the period of time allocated to him or her upon his or her log-in to the system, thereby allowing the user to formulate an efficient task plan. In addition, the task schedule screen display function allows the user to check that none of the tasks are left undone when he or she logs out from the system.

A task notification setting field 704 is used to set whether or not to give a notification display that prompts the user to perform a task when an occurrence time of day of each task event arrives or a reserved time of day when the user so sets arrives. A task notification time setting field 705 is used to set a time at which an advance task notification is performed.

A collective task notification setting field 706 and a collective task notification time setting field 707 are used to set, when a first task event encounters a task time of day, whether or not to give a notification display for recommending collective performance of tasks that encounter respective task times of day within a predetermined period of time that begins with the task time of day of the first task event.

An advance task notification before allocated time end setting field 708 and an advance task notification before allocated time end time setting field 709 are used to set to notify residual tasks before a predetermined period of time of an allocated time end arrives. The foregoing task notification functions prompt the user to perform tasks, thus preventing omission of a task.

The times set in the task notification setting field 704, the task notification time setting field 705, the collective task notification setting field 706, the collective task notification time setting field 707, the advance task notification before allocated time end setting field 708, and the advance task notification before allocated time end time setting field 709 are controlled by the notifying section 114 shown in Fig. 2. When a reserved time arrives, the notifying section 114 notifies the user of performance of a corresponding task using the task notifying dialog screen 904 to be described later with reference to Fig. 9.

A work schedule host system reception setting field 710 is used to set whether or not to receive the work schedule information required for creating the task schedule information from the host system connected to the network. A task schedule host system transmission setting field 711 is used to set whether or not to transmit the created task schedule information to the host system.

Providing the foregoing functions to communicate with the host system enables information to be shared between the host system and the automatic analyzer, thus allowing the host system to efficiently provide integrated control of a plurality of automatic analyzers and a plurality of users in large-scale hospitals and testing facilities.

The information input through the task schedule control setting screen 701 is saved by depression of a set button 712.

The following describes with reference to Fig. 8 steps of task schedule preparing and control processes in the automatic analyzer according to the embodiment of the present invention.

Fig. 8 is a flowchart showing steps of the task schedule preparing and control processes in the automatic analyzer according to the embodiment of the present invention.

In step 801, it is determined whether a setting is made to receive a work schedule from the host system. If the setting is made, the work schedule information is received from the host system connected to the network in step 802.

In step 803, work schedule information is read. If it is determined in step 801 that the setting is not made, the work schedule information registered in the work schedule input screen on the operator unit PC is to be read.

Then in step 804, analyzer task event information relating to the reagent, calibration, accuracy control, and maintenance tasks that can be predicted to occur from parameter and reagent information registered in the analyzer and measurement and maintenance records is read.

In step 805, a task schedule for each person in charge is prepared on the basis of the work schedule information read in step 803 and by incorporating the analyzer task event information read in step 804. The steps up to step 805 constitute a process for preparing the task schedule.

Subsequent steps constitute a process for controlling the task schedule. In step 806, it is determined whether there is any task event having the notification time of day arrived. If it is determined that there is such a task event, it is then determined in step 807 whether there is any task event having the notification time of day arriving within a specified period of time. If it is determined that there is such a task event, the user is notified in step 808 to perform applicable tasks collectively. If it is determined that there is no such task events, the user is notified in step 809 to perform the individual task.

Exemplary cases in which it is appropriate to perform tasks collectively include the following. For example, with respect to the reagent addition task, timing at which to add the reagent depends on the amount of reagent still available for use, thus varying from one reagent to another. Assume, however, a case, for example, in which a task event for adding a second reagent is scheduled to occur 10 minutes after a task event for adding a first reagent. Labor of reagent addition can be saved if the addition of the first reagent and the addition of the second reagent are successively performed. It is determined to be appropriate to perform tasks collectively in such a case.

It is common practice to perform calibration at predetermined time intervals for fear of deterioration of the reagent. In contrast, accuracy control is typically performed each time a predetermined number of samples have been measured. In a case, for example, in which the calibration and the accuracy control for a particular reagent are scheduled to occur at different timings apart slightly from each other, performing the calibration and the accuracy control in sequence is more efficient. In such a case, it is determined to be appropriate to perform the tasks collectively.

Then, in step 810, it is determined whether a set time of the end of an allocated period of time is yet to arrive. If it is determined that the set time is yet to arrive, the user is notified in step 811 to perform task events yet to be performed within the allocated period of time as residual tasks.

In step 812, it is determined whether there is any task that was not performed within the allocated period of time. If it is determined that there is such a task, the residual task is carried over to the task schedule for the subsequent person in charge in step 813.

Finally in step 814, it is determined whether there is any setting made to transmit the task schedule to the host system. If it is determined that there is such a setting made, the task schedule information is transmitted to the host system in step 815.

The following describes with reference to Fig. 9 an exemplary task notifying dialog screen in the automatic analyzer according to the embodiment of the present invention.

Fig. 9 is an explanatory drawing illustrating an exemplary task notifying dialog screen in the automatic analyzer according to the embodiment of the present invention.

When a task performance time of day arrives, the task notifying dialog screen 904 appears to prompt the user to perform the task. Fig. 9 represents a case in which the task notifying dialog screen 904 is displayed while the user is using a patient sample measurement screen 901.

The task notifying dialog screen 904 displays details of a specific task prompted to be performed in a task detail display area 905. A plurality of tasks, if involved, is displayed in a list format. Depression of a task performance screen shift button 906 displayed for each task causes the screen to shift directly to each task performance screen for performing the corresponding task, allowing the task to be quickly performed.

Depression of a task schedule screen shift button 907 causes the screen to shift to the task schedule screen, allowing the task schedule to be confirmed.

Depression of a close button 908 closes the task notifying dialog screen.

Providing the task notification function as described above allows the user to be notified of a task at a scheduled time of day even when he or she is performing another task, thus preventing omission of a task.

A task schedule screen shift button 903 is used to display the task schedule screen. Preferably, the task schedule screen shift button 903 is disposed in a global screen-independent display area 902 that is not dependent on the display of each screen such that the task schedule screen can be accessed and displayed from any other screen.

As described heretofore, in the embodiment, the advance input of the user's work schedule allows the time-series task schedule chart that represents a list of tasks to be performed by the user within the period of time allocated to him or her to be displayed when the user logs in to the operator unit of the automatic analyzer. The user can thereby check the specific task to be performed at the start of work, thus being able to formulate an efficient task plan in line with his or her work schedule.

In addition, the function of automatically notifying the user of the scheduled time of day for a task and of a task yet to be performed to thereby prompt the user to perform the tasks and the function of carrying a task yet to be performed over to the subsequent person in charge prevent omission of tasks. Additionally, an inquiry about task schedules of the preceding and subsequent persons in charge enables a task performance status to be confirmed and a task to which the person in charge is not accustomed to be performed ahead of the schedule.

Additionally, providing the communication function that receives information on the user's work schedule from the host system that integrally controls a plurality of automatic analyzers connected over the network and transmits information on the prepared task schedule to the host system enables the host system to integrally control the task schedule encompassing tasks to be performed by the user for the automatic analyzers within the period of time allocated to him or her. A workload on the user can thus be lessened and an efficient control of the task schedule can be achieved in a large-scale testing facility.

### Description of Reference Symbols

100 Operator unit PC
110 Control unit
120 Input unit
130 Display unit
140 Storage unit
112 Planning section
113 Schedule preparing section
114 Notifying section
116 Reserving section
118 Determining section
AA Analyzing unit
HB Hub
RED Reagent disk
REG Reagent
RSY Remote system
SAI Sample loading unit
SAO Sample storing unit
SML Sample rack
SMP Sample
TRL Transfer line
USR User
USY Host system
301 Task schedule screen
302 Log-in user
303 Person-in-charge advance/retreat buttons
304 Time advance/retard buttons
305 Schedule display area
306 Allocated time frame
307 Current time of day line
308 Reagent task display area
309 Reagent task event
310 Calibration task display area
311 Calibration task event
312 Accuracy control task display area
313 Accuracy control task event
314 Maintenance task display area
315 Maintenance task event
316 Task detail display area
317 Task reserving button
318 Task performance screen shift button
401, 402 Reagent task event
403 to 406 Calibration task event
407 to 410 Accuracy control task event
411, 412 Maintenance task event
413 Task detail display area
502 Work schedule input screen
503 Work schedule input process
504 Work schedule information
505 Analyzer task even control process
506 Analyzer task event information
507 Task schedule preparing process
508 Task schedule information
509 Task schedule screen
510 Each task performance screen
601 Work schedule input screen
602 Calendar
603 Schedule input area
604 Person in charge input area
605 Schedule display area
606 Personnel assignment display area
607 Register button
701 Task schedule control setting screen
702 Log-in task schedule display setting
703 Log-out task schedule display setting
704 Task notification setting
705 Task notification time setting
706 Collective task notification setting
707 Collective task notification time setting
708 Advance task notification before allocated time end setting
709 Advance task notification before allocated time end time setting
710 Work schedule host system reception setting
711 Task schedule host system transmission setting
712 Set button
801 to 815 Flowchart steps
901 Patient sample measurement screen
902 Screen-independent display area
903 Task schedule screen shift button
904 Task notifying dialog screen
905 Task detail display area
906 Task performance screen shift button
907 Task schedule screen shift button
908 Close button

## Claims

1. An automatic analyzer comprising:
an input unit (120) for inputting a work schedule for the user of the automatic analyzer;
a planning section (112) adapted to schedule in advance task events relating to reagent, calibration, accuracy control and maintenance, the task events being predictable to occur from parameter and reagent information registered in the automatic analyzer and measurement and maintenance records;
a schedule preparing section (113) adapted to prepare, from the task events scheduled by the planning section (112), a time-series task schedule representing a list of tasks to be performed by the user within a period of time allocated to him or her;
a reserving section (116) adapted to allow the user to set any scheduled performance time of day for a task event on the task schedule; and
a display unit (130) adapted to display the task schedule prepared by the schedule preparing section,
**characterised in that**
the work schedule includes a test schedule with specific times of day specified for tests,
the schedule preparing section (113) is adapted to prepare the task schedule further from the work schedule input from the input unit (120) and prepare the task schedule as a time-series task schedule chart (308~315) representing said tasks to be performed by the user, wherein the display unit (130) is adapted to display the time-series task schedule chart (308~315) on a screen (301) together with a schedule area (305) including the test schedule with the specific times of day specified for tests, and to automatically display, upon a log-in to, or a log-out from, a system by a user, the task schedule for the user for the corresponding time slot and to display a task not performed by a person previously in charge so as to be carried over to the logged-in user.

2. The automatic analyzer according to claim 1, further comprising:
a first notifying section (114) for prompting the user to perform the task when the time of day reserved by the reserving section (116) arrives; and
a task performance screen shift button (906) for directly shifting to an operating screen for performing a task.

3. The automatic analyzer according to claim 1, further comprising:
a determining section (118) for recognizing the task performed on the automatic analyzer to thereby automatically determine whether a task event displayed on the task schedule has been performed or has yet to be performed, wherein
the display unit (130) is adapted to display on the task schedule whether or not a task has been performed.

4. The automatic analyzer according to claim 3, further comprising:
a second notifying section for prompting, when a set time of the end of an allocated period of time arrives, the user to perform a task event yet to be performed.

5. The automatic analyzer according to claim 2, wherein the first notifying section (114) is adapted to notify a user of a recommendation that a plurality of tasks occurring within a predetermined period of time be performed collectively.

6. The automatic analyzer according to claim 1, the automatic analyzer being connected to a host system (USY) adapted to store information on a work schedule of operators, wherein the automatic analyzer is adapted to acquire the information on the work schedule from the host system.

7. The automatic analyzer according to claim 1, adapted to transmit information on the prepared task schedule to a host system (USY).

## Patentansprüche

1. Automatischer Analysator umfassend:
eine Eingabeeinheit (120) zum Eingeben eines Arbeitsplans für den Benutzer des automatischen Analysators;
einen Planungsabschnitt (112), um vorab Reagenz, Kalibrierung, Genauigkeitskontrolle und Wartung betreffende Aufgabenereignisse zu planen, die anhand von Messungen und Wartungsprotokollen sowie im automatischen Analysator registrierten Reagenzinformationen und Parametern voraussichtlich auftreten können;
einen Planerstellungsabschnitt (113), um aus den vom Planungsabschnitt (112) geplanten Aufgabenereignissen einen Zeitfolge-Aufgabenplan zu erstellen, der eine Liste von Aufgaben darstellt, die von einem Benutzer innerhalb einer ihm oder ihr zugewiesenen Zeitspanne auszuführen sind;
einen Reservierungsabschnitt (116), um dem Benutzer zu erlauben, für ein Aufgabenereignis auf dem Aufgabenplan eine geplante Ausführungs-Tageszeit festzulegen; und
eine Anzeigeeinheit (130), um den vom Planerstellungsabschnitt erstellten Aufgabenplan anzuzeigen,
**dadurch gekennzeichnet, dass**
der Arbeitsplan einen Testplan mit bestimmten Tageszeiten enthält, die für Tests bestimmt sind, und
der Planerstellungsabschnitt (113) eingerichtet ist, den Aufgabenplan außerdem aus dem von der Eingabeeinheit (120) her eingegebenen Arbeitsplan zu erstellen und den Aufgabenplan als Zeitfolge-Aufgabenplan-Diagramm (308-315) zu erstellen, das die vom Benutzer auszuführenden Aufgaben darstellt, wobei die Anzeigeeinheit (130) eingerichtet ist, das Zeitfolge-Aufgabenplan-Diagramm (308-315) auf einem Bildschirm (301) zusammen mit einem Planbereich (305) anzuzeigen, der den Testplan mit den bestimmten Tageszeiten enthält, die für Tests bestimmt sind, und beim Login oder Logout eines Benutzers in das oder aus dem System automatisch den Aufgabenplan für den Benutzer für das entsprechende Zeitfenster anzuzeigen und eine von einer zuvor zuständigen Person nicht ausgeführte Aufgabe auf den eingeloggten Benutzer übertragen anzuzeigen.

2. Automatischer Analysator nach Anspruch 1, außerdem umfassend:
einen ersten Benachrichtigungsabschnitt (114), um den Benutzer aufzufordern, die Aufgabe auszuführen, wenn die vom Reservierungsabschnitt (116) reservierte Tageszeit erreicht wird; und
einen Aufgabenausführungsschirm-Wechselknopf (906), um direkt zu einem Arbeitsschirm zum Ausführen einer Aufgabe zu wechseln.

3. Automatischer Analysator nach Anspruch 1, außerdem umfassend:
einen Bestimmungsabschnitt (118), um die am automatischen Analysator ausgeführte Aufgabe zu erkennen und dadurch automatisch zu bestimmen, ob ein auf dem Aufgabenplan angezeigtes Aufgabenereignis ausgeführt worden ist oder noch auszuführen ist, wobei
die Anzeigeeinheit (130) eingerichtet ist, auf dem Aufgabenplan anzuzeigen, ob die Aufgabe ausgeführt worden ist.

4. Automatischer Analysator nach Anspruch 3, außerdem umfassend:
einen zweiten Benachrichtigungsabschnitt, um einen Benutzer aufzufordern ein noch auszuführendes Aufgabenereignis auszuführen, wenn eine festgelegte Zeit für das Ende einer zugewiesenen Zeitspanne erreicht wird.

5. Automatischer Analysator nach Anspruch 2, wobei der erste Benachrichtigungsabschnitt (114) eingerichtet ist, einen Benutzer über eine Empfehlung zu benachrichtigen, dass mehrere innerhalb einer vorbestimmten Zeitspanne auftretende Aufgaben gemeinsam auszuführen sind.

6. Automatischer Analysator nach Anspruch 1, der mit einem Hostsystem (USY) verbunden ist, das zum Speichern von Informationen über einen Arbeitsplan von Bedienern eingerichtet ist, wobei der automatische Analysator eingerichtet ist, vom Hostsystem die Informationen über den Arbeitsplan zu gewinnen.

7. Automatischer Analysator nach Anspruch 1, der zum Übermitteln von Informationen über den vorbereiteten Arbeitsplan an ein Hostsystem (USY) eingerichtet ist.

## Revendications

1. Analyseur automatique comprenant :
une unité (120) d'entrée pour entrer un programme de travail pour l'utilisateur/trice de l'analyseur automatique ;
une section (112) de planification adaptée à programmer à l'avance des événements de tâches se rapportant à un réactif, un étalonnage, un contrôle de précision et une maintenance, les événements de tâches prévisibles de survenir à partir d'informations de paramètres et de réactifs enregistrées dans l'analyseur automatique et de registres de mesures et de maintenance ;
une section (113) de préparation de programme adaptée à préparer, à partir des événements de tâches programmés par la section (112) de planification, un programme chronologique de tâches représentant une liste de tâches devant être exécutées par l'utilisateur/trice au sein d'une période de temps attribuées à lui ou elle ;
une section (116) de réservation adaptée à permettre à l'utilisateur/trice de fixer une quelconque heure du jour d'exécution programmée pour un événement de tâche sur le programme de tâches ; et
une unité (130) d'affichage adaptée à afficher le programme de tâches préparé par la section de préparation de programme,
**caractérisé en ce que**
le programme de travail inclut un programme de tests avec des heures du jour spécifiques spécifiées pour des tests,
la section (113) de préparation de programme est adaptée à préparer le programme de tâches en outre à partir du programme de travail entré depuis l'unité (120) d'entrée et préparer le programme de tâches comme un tableau (308~315)de programme chronologique de tâches représentant lesdites tâches devant être exécutées par l'utilisateur/trice, dans lequel l'unité (130) d'affichage est adaptée à afficher le tableau (308~315)de programme chronologique de tâches sur un écran (301) ensemble avec une zone de programme (305) incluant le programme de tests avec les heures du jour spécifiques spécifiées pour des tests, et à afficher automatiquement, lors d'une connexion à un, ou d'une déconnexion d'un système par un(e) utilisateur/trice, le programme de tâches pour l'utilisateur/trice pour l'intervalle temporel correspondant et à afficher une tâche non exécutée par une personne précédemment en charge de façon à ce qu'elle soit transférée à l'utilisateur/trice connecté.

2. Analyseur automatique selon la revendication 1, comprenant en outre :
une première section (114) de notification pour inviter l'utilisateur/trice à exécuter la tâche lorsque l'heure du jour réservée par la section (116) de réservation arrive ; et
un bouton (906) de changement d'écran d'exécution de tâches pour directement passer à un écran d'opération pour exécuter une tâche.

3. Analyseur automatique selon la revendication 1, comprenant en outre :
une section (118) de détermination pour reconnaître la tâche exécutée sur l'analyseur automatique pour ainsi déterminer automatiquement si un événement de tâche affiché sur le programme de tâches a été exécuté ou reste encore à être exécuté, dans lequel
l'unité (130) d'affichage est adaptée à afficher sur le programme de tâches si une tâche a ou non été exécutée.

4. Analyseur automatique selon la revendication 3, comprenant en outre :
une deuxième section de notification pour inviter, lorsqu'une heure fixée de la fin d'une période temps attribuée arrive, l'utilisateur à exécuter un événement de tâche restant encore à être exécuté.

5. Analyseur automatique selon la revendication 2, dans lequel la première section (114) de notification est adaptée à notifier à un(e) utilisateur/trice une recommandation qu'une pluralité de tâches survenant à l'intérieur d'une période de temps prédéterminée soient exécutées collectivement.

6. Analyseur automatique selon la revendication 1, l'analyseur automatique étant connecté à un système hôte (USY) adapté à stocker des informations sur un programme de travail d'opérateurs, dans lequel l'analyseur automatique est adapté à acquérir les informations sur le programme de travail depuis le système hôte.

7. Analyseur automatique selon la revendication 1, adapté à transmettre des informations sur le programme de tâches préparé à un système hôte (USY).
